# EUROPEAN PATENT SPECIFICATION

(11) **EP 4 295 789 B1**
(45) Date of publication and mention of the grant of the patent: **30.07.2025**
(21) Application number: 23209721.2
(22) Date of filing: 03.03.2020
(51) Int. Cl.: A61B 17/221, A61B 17/22, A61B 17/12

(54) **FUNNEL ACTUATION SYSTEMS**
TRICHTERBETÄTIGUNGSSYSTEME
SYSTÈMES D'ACTIONNEMENT D'ENTONNOIR

(30) Priority: 04.03.2019 US 201962813718 P
(43) Date of publication of application: 27.12.2023
(62) Divisional of application: 20160688.6
(73) Proprietor: Neuravi Limited, Galway H91 K5YD (IE)
(72) Inventor: KELLY, Ronald, Galway (IE); KEATING, Karl, Galway (IE)
(74) Representative: Carpmaels & Ransford LLP

(56) References cited:
- AU-B2- 2015 271 876
- US-A- 5 102 415
- US-A1- 2002 022 859
- US-A1- 2005 159 770
- US-A1- 2009 163 846
- US-A1- 2010 030 256
- US-A1- 2010 137 846
- US-A1- 2013 225 934
- US-A1- 2017 100 142
- US-A1- 2019 021 759
- US-A1- 2019 046 219
- US-B1- 10 172 634

## Description

### Field of the Disclosure

The present disclosure generally relates to devices and methods for removing blockages from blood vessels during intravascular medical treatments. More specifically, the present disclosure relates to clot retrieval devices comprising an expandable and collapsible funnel catheter.

### Background

Clot retrieval catheters and devices are used in mechanical thrombectomy for endovascular intervention, often in cases where patients are suffering from conditions such as acute ischemic stroke (AIS), myocardial infarction (MI), and pulmonary embolism (PE). Accessing the neurovascular bed in particular is challenging with conventional technology, as the target vessels are small in diameter, remote relative to the site of insertion, and are highly tortuous. Traditional devices are often either too large in profile, lack the deliverability and flexibility needed to navigate tortuous vessels, difficult to administer and use, or are not effective at removing a clot when delivered to the target site.

Conventional clot retrieval catheters suffer from a number of drawbacks. First, the diameters of catheters themselves must be small enough to avoid causing significant discomfort to the patient. The retrieval catheter must also be sufficiently flexible to navigate the vasculature and endure high strains, while also having the axial stiffness to offer smooth advancement along the route. Once at the target site, typical objects to be retrieved from the body are substantially larger in size than the catheter tip, making it more difficult to retrieve objects into the tip. For example, firm, fibrin-rich clots can often be difficult to extract as they can become lodged in the tip of traditional fixed-mouth catheters. Additionally, this lodging can cause softer portions to shear away from the firmer regions of the clot.

Small diameters and fixed tip sizes are also less efficient at directing the aspiration necessary to remove blood and thrombus material during the procedure. Fixed tip sizes can cause a clot to shear or break apart as the clot enters the tip opening. The suction must be strong enough such that any fragmentation that may occur as a result of aspiration or the use of a mechanical thrombectomy device can be held stationary so that fragments cannot migrate and occlude distal vessels. However, when aspirating with a fixed-mouth catheter, a significant portion of the aspiration flow ends up coming from vessel fluid proximal to the tip of the catheter, where there is no clot, because the diameter of the funnel catheter is smaller than that of the vessel. This significantly reduces aspiration efficiency, lowering the success rate of clot removal.

Any catheter design attempting to overcome these challenges with an expanding distal tip or structure would need to have the strength to extract the clot and exert a steady radial force in the expanded state. The same structure would also need to be sufficiently flexible and elastic to survive the severe mechanical strains imparted when navigating tortuous vasculature when in a collapsed state.

US2017/0100142A1 describes systems and methods for management of thrombosis.

US 2005/0159770 A1 discloses a retrieval catheter having a distal funnel sheath and a control wire to adjust the tension on a distal expandable ring.

The present design is aimed at providing an improved retrieval catheter which addresses the above-stated deficiencies.

### Summary

The invention is defined by claim 1. Embodiments of the invention are defined by the dependent claims. It is an object of the present design to provide devices and methods to meet the above-stated needs. It is therefore desirable for a clot retrieval catheter device to have a component with a large-mouth clot-facing expandable tip for removal and easy retrieval of the clot while also having a collapsed state that is low-profile and sufficiently flexible for deliverability to the target site.

According to the present disclosure, there is provided a device which can have a funnel catheter having an expandable distal tip. The funnel catheter can originally be placed in a collapsed configuration for delivery through vasculature to a target thrombus location. Once positioned, the expandable tip can be radially expanded to create a funnel for aspirating or retrieving clots from a vessel. The funnel catheter can be at least partially enclosed by an outer sheath for purposes of delivery to the target site or to assist in expansion of the funnel catheter tip.

The outer sheath can facilitate the introduction of dilators, funnels, microcatheters, guidewires, or any of a number of commercially available products to a target site within the vasculature. The funnel catheter may be pre-loaded into the outer sheath prior to the administration of the device to a target site.

The device can have an outer sheath positioned within the vasculature of a patient so that the distal end is proximate a targeted occlusive clot. The funnel catheter can be located at least partially within the outer sheath, and aspiration can be directed through the funnel catheter. The device can be advanced through the vasculature in a collapsed configuration. The expanding distal tip can grow radially outward to contact, and form a seal with, the inner wall of a blood vessel to aid in aspirating or receiving a clot.

The funnel catheter can be used in conjunction with other mechanical devices for the removal of clots. In an example, a dilator can be at least partially enclosed within the funnel catheter. The dilator and funnel catheter can be advanced to a target aspiration location over a guidewire. The distal tip can be restrained in a collapsed delivery configuration by a funnel restraining sheath attached to the dilator during administration of the device to the target site. The distal tip can be sized and configured such that when it is deployed at the target site and expanded to a maximum diameter, the tip can expand to atraumatically contact the inner vessel walls to provide the maximum possible opening for aspirating or otherwise dislodging and receiving a clot. The distal tip can be exposed from within the funnel retaining sheath by advancing the dilator distally away from the funnel catheter, or by retracting the funnel proximally relative to the dilator. The distal tip can then grow radially outward to contact, and form a seal with, the inner wall of a blood vessel. The clot can then be extracted by the device once the dilator has been fully removed.

In an example, a device can have an outer sheath and a funnel catheter within the outer sheath in the collapsed configuration as the device is positioned proximate a target clot location. The funnel catheter can further be at least partially enclosed within a funnel sheath inside the outer sheath, and at least a portion of the distal end of the funnel catheter can extend distally into the outer sheath from the funnel sheath. Once the device is in position near the target location, the outer sheath can be retracted to expose the funnel catheter. The distal tip can then radially expand to seal the vessel in preparation for removing a clot. The funnel catheter can also be advanced distally from the outer sheath after expansion of the distal tip to maintain a better seal with the blood vessel or create more efficient access to a clot. The distal tip can invert with low force during advancement to avoid injuring the blood vessel, and revert to its funnel shape when retracted. Once the distal tip is deployed and appropriately positioned, the clot can be aspirated or removed.

In some examples, the distal end of the funnel catheter can expand via a pull wire. Through use of the pull wire, the device might not require an outer sheath to expand or collapse the distal tip, although an outer sheath may still be utilized for delivery of the device to the treatment site. The funnel catheter can have an outer lamination layer covering at least a portion of the funnel catheter and with openings formed in the layer that allow translation of one or more pull wires. The funnel catheter can also have a retaining sheath attached to the catheter distally of the lamination layer via the one or more pull wires. When positioned at a target location, the distal tip can be expanded to the deployed configuration by utilizing the one or more pull wires to shift the floating retaining sheath proximally towards the outer lamination layer, thereby exposing the distal tip and allowing it to expand to a predisposed diameter and make contact with a vessel wall. Once the distal tip is deployed and appropriately positioned, the clot can be aspirated or removed.

In an example, the funnel catheter can contain ferromagnetic material at the distal end, including in the distal tip, and the funnel catheter can be expanded to the deployed state or retracted to the collapsed state by passing it over an inner dilator wherein at least a portion of the inner dilator can have magnetic properties. The dilator can be at least partially enclosed within the funnel catheter. If the ferromagnetic distal end passes over a magnetic portion of dilator, the ferromagnetic material at the distal end can be attracted to the magnetic portion of the dilator which can retract the funnel catheter into the collapsed configuration. The restraining force between the ferromagnetic funnel and magnetic dilator can be removed by retracting the dilator relative to the funnel catheter, which can break the alignment between the ferromagnetic distal end of the funnel catheter and the magnetic portion of the dilator. This allows the distal end to expand to the deployed funnel configuration. Once the distal tip is deployed and appropriately positioned within a blood vessel, a clot can be aspirated or removed after removal of the dilator.

In an example, the funnel catheter can be supplied to a target site in a collapsed configuration for low-profile atraumatic delivery to the target site. The distal tip of the funnel catheter can have a wider braid angle and/or a lower pics-per-inch (PPI) than areas of the funnel catheter proximal to the tip. (1 inch ≈ 2.54 cm).

The distal tip can be encapsulated or covered with a highly elastic material or membrane. When aspiration is applied to the proximal end of the funnel catheter, a negative flow can be achieved at the distal tip. The distal tip can then be compressed proximally as a result of the compressive forces applied through negative flow. As a result, the diameter of the distal tip can increase upon proximal compression and the membrane stretched over the tip can expand as well. The distal tip can expand to contact the vessel walls at the target site, sealing the vessel and forming a funnel mouth that can receive a clot. When aspiration flow is stopped or removed, the shape memory of the elastic membrane and/or distal tip can return to the collapsed configuration. When the distal tip returns to the collapsed configuration, it can grasp and retain any portions of the clot lodged therein.

In an example, a zip cover actuation and a pull wire can be utilized to expand the distal tip to the deployed state for clot removal. The device can have a funnel catheter, predisposed to expand outwardly at the distal tip upon deployment, with an outer lamination layer covering at least a portion of the funnel catheter and with openings formed in the layer that allow translation of one or more pull wires. The funnel catheter can also have a retaining sheath located distally to the lamination layer and attached to the catheter via the one or more pull wires. The retaining sheath can include a longitudinal separation. The longitudinal separation can have a series of openings on either side of the separation, the openings being aligned longitudinally. The pull wire can extend through the openings, holding the retaining sheath in a configuration to maintain the distal tip in the collapsed configuration. The pull wire can be retracted to break the longitudinal connections of the retaining sheath, allowing the distal tip to expand. Once the distal tip is deployed and appropriately positioned within a blood vessel, a clot can be aspirated and removed.

In an example, the funnel catheter can be at least partially encapsulated by an outer sheath and can expand radially upon deployment due to one or more inner support structures that can flare radially outward when the outer sheath is retracted. The flaring of the internal support structures can expand the distal tip to atraumatically contact a vessel wall, creating a funnel for clot aspiration. Once the distal tip is deployed and appropriately positioned, the clot can be aspirated. The retraction force on the outer sheath can later be released to move the outer sheath distally relative to the funnel catheter, re-covering the distal tip and collapsing the distal tip back into the collapsed configuration for removal of the device from the vasculature or further advancement of the device into the vasculature of the patient.

An example method for removing a clot can include one or more of the following steps presented in no particular order, and the method can include additional steps not included here. The method can include accessing an arterial blood vessel of a patient, advancing a device including a funnel catheter with an expandable distal tip into the patient's vasculature, expanding the distal tip the funnel catheter proximate a target thrombus; stimulating the thrombus into the mouth of the funnel catheter; and retrieving the funnel catheter with the captured thrombus through the vasculature and out of the patient.

Other aspects and features of the present disclosure will become apparent to those of ordinary skill in the art, upon reviewing the following detailed description in conjunction with the accompanying figures.

### Brief Description of the Drawings

The above and further aspects of this disclosure are further discussed with the following description of the accompanying drawings, in which like numerals indicate like structural elements and features in various figures. The drawings are not necessarily to scale, emphasis instead being placed upon illustrating principles of the disclosure. The figures depict one or more implementations of the inventive devices, by way of example only, not by way of limitation. It is expected that those of skill in the art can conceive of and combining elements from multiple figures to better suit the needs of the user.
Figs. 1a-1e are illustrations of a retrieval aspiration catheter device including an outer sheath layer and a funnel catheter in a treatment sequence to retrieve a clot according to aspects of the present disclosure;
Figs. 2a-2e are illustrations of a retrieval aspiration catheter device including an outer sheath layer and a funnel catheter in a treatment sequence to retrieve a clot according to aspects of the present disclosure;
Figs. 3a-3d are illustrations of the expansion of the distal tip of the funnel catheter in an exemplary treatment device according to aspects of the present invention;
Figs. 4a-4b are illustrations of the expansion of the distal tip of the funnel catheter in an exemplary treatment device according to aspects of the present disclosure;
Figs. 5a-5c are illustrations of the expansion of the distal tip of the funnel catheter in an exemplary treatment device according to aspects of the present disclosure;
Figs. 6a-6e are illustrations of the expansion of the distal tip of the funnel catheter in an exemplary treatment device according to aspects of the present invention;
Figs. 7a-7c are illustrations of the expansion of the distal tip of the funnel catheter in an exemplary treatment device according to aspects of the present disclosure;
Fig. 8 is a flow diagram outlining a method for clot removal according to aspects of the present disclosure.

### Detailed Description

Specific examples of the present disclosure are now described in detail with reference to the Figures, where identical reference numbers indicate elements which are functionally similar or identical. The examples address many of the deficiencies associated with traditional catheters, such as inefficient clot removal and inaccurate deployment of catheters to a target site.

Accessing the various vessels within the vascular, whether they are coronary, pulmonary, or cerebral, involves well-known procedural steps and the use of a number of conventional, commercially-available accessory products. These products, such as angiographic materials and guidewires are widely used in laboratory and medical procedures. When these products are employed in conjunction with the system and methods of this disclosure in the description below, their function and exact constitution are not described in detail.

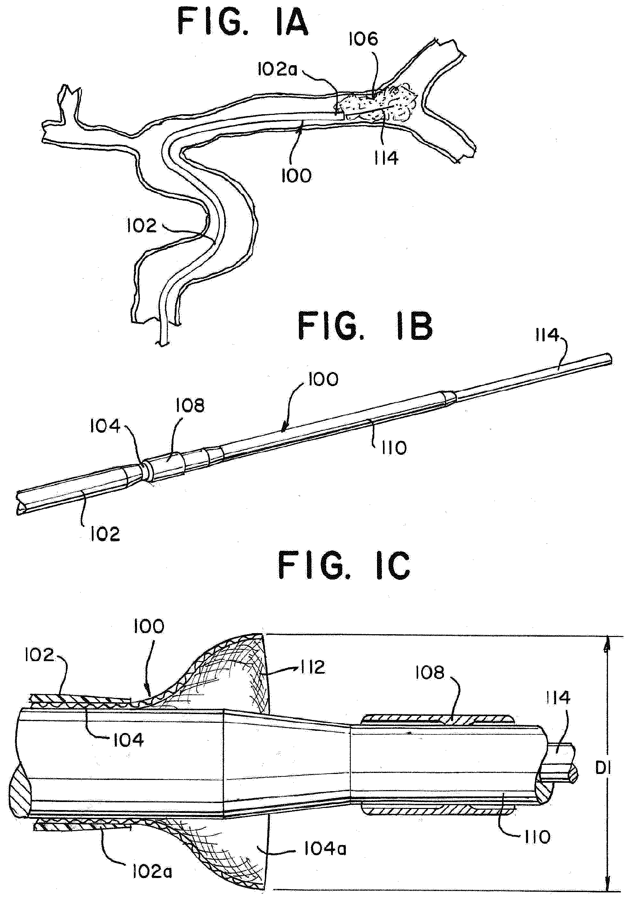

Referring to the figures, in Fig. 1a there is illustrated a device **100** for removing an occlusive clot from a vessel of a patient according to this disclosure. The device **100** can have an outer sheath layer **102** facilitating the introduction of microcatheters, funnel catheters, guidewires, or any of a number of other products to a target site within the vasculature. In some examples, the outer sheath **102** can be one or any of a guide catheter, sheath catheter, or an intermediate catheter. The outer sheath **102** can have a distal end **102a,** a proximal end located outside of a patient's vasculature, and an internal lumen extending proximal of the distal end **102a** and terminating within the proximal end. The outer sheath **102** can be positioned within the vasculature of a patient so that the distal end **102a** is proximate a targeted occlusive clot **106.**

As illustrated in Figs. 1b and 1c, the device **100** can also have a funnel catheter **104** with an expanding distal tip **112.** The funnel catheter **104** can be located at least partially within the lumen of the outer sheath **102.** The funnel catheter **104** can have a diameter of approximately 1.8mm-2.0mm in the collapsed configuration. The funnel catheter **104** may be pre-loaded into the outer sheath **102** prior to the administration of the device **100** to a target site. The funnel catheter **104** as depicted in Fig. 1b is in a collapsed configuration for delivery of the device **100** to a target location.

As in Fig. 1c, the funnel catheter **104** may have a proximal end located outside a patient's vasculature, a distal end **104a,** and an internal lumen extending proximal of the distal end **104a** and terminating at the proximal end. The lumen can be defined by a tubular support, and can be configured for the passage of guidewires, microcatheters, stent retrievers, and other such devices therethrough. The lumen can also direct aspiration from the proximal end of the funnel catheter to the distal tip **112** of the funnel catheter **104.** The proximal end of the funnel catheter **104** can include at least one aperture for the connection or removal of ancillary devices. The proximal end of the funnel catheter **104** can include an aperture for aspiration flow or contrast injection.

The distal section **104a** of the funnel catheter **104** can have good push and trackability characteristics to aid in advancing it to the target location. It can therefore have multiple designs, or be fabricated from multiple materials, to give a reducing stiffness profile along the length to minimize insertion and retraction forces, and to enhance torqueability, pushability, flexibility, and kink resistance of the funnel catheter **104.** Features can also be incorporated which bias bending about certain planes or encourage twisting for ease of delivery to a target site.

The funnel catheter **104** can be used in conjunction with separate mechanical devices for the removal of clots. One such example device, as illustrated in Figs. 1b an 1c, can include a dilator **110** at least partially enclosed within the lumen of the funnel catheter **104.** The dilator **110** can be a polymer tube catheter used in conjunction with a balloon guide catheter or a long sheath. The dilator **110** towards its distal end can have an attached distal funnel retaining sheath **108.** The funnel retaining sheath **108** can be in the form of an umbrella membrane. At least a portion of the distal end **104a** of the funnel catheter **104** can be collapsed and fitted inside the distal funnel retaining sheath **108** where the distal funnel retaining sheath **108** exhibits enough resistance to the radial expansion force of the distal tip **112** of the funnel catheter **104.** The dilator **110** and funnel catheter **104** can be advanced to a target aspiration location over a guidewire **114.** The funnel retaining sheath **108** assures a smooth crossing profile and securement of the funnel catheter **104** during delivery. The dilator **110** can be pre-loaded into the funnel catheter **104** prior to the administration of the device **100** to a target site. The funnel catheter **104** can be wrapped or prolapsed inside the distal funnel retaining sheath **108** of the dilator **110** during delivery. The dilator **110** can have a proximal end and a distal end, and the dilator **110** can have a distally reducing diameter along the length of the dilator **110** such that the diameter of the dilator at the proximal end of the dilator is greater than the diameter of the dilator at the distal end.

The distal tip **112** can be predisposed to open to a diameter **D1** when in a radially expanded deployed configuration in which the tip assumes a substantially conical or funnel shape. The distal tip can be restrained in a collapsed delivery configuration by a funnel restraining sheath **108** attached to the dilator **110** during administration of the device **100** to a target site. In the collapsed state, the tip **112** can have a radial dimension less than a maximum radial dimension of the tip **D1.** In some devices, the tip **112** can have a radial dimension **D1** of less than approximately 5.5mm. In some devices, the tip **112** can have a radial dimension **D1** of less than approximately 3.5mm. The distal tip **112** can be sized and configured such that when deployed at the target site and expanded to diameter **D1,** it expands to atraumatically contact inner blood vessel walls to provide the maximum possible opening for aspirating or otherwise dislodging and receiving the clot. The expanded tip **112** can also prevent the unwanted aspiration of blood proximal to the tip **112.**

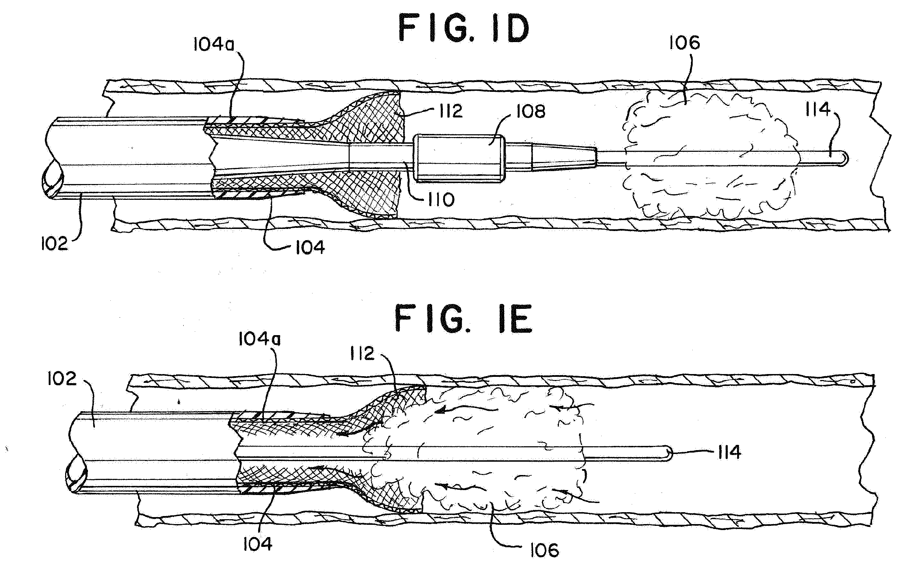

As depicted in Fig. 1d, when expanding, the tip **112** can first be exposed from within the funnel retaining sheath **108** by displacing the dilator **110** relatively distally of the funnel catheter **104.** This can be accomplished by advancing the dilator **110** distally of the funnel catheter **104,** or by retracting the funnel catheter **104** proximally relative to the dilator **110.** The distal tip **112** can then grow radially outward. As illustrated in Fig. 1e, in the expanded state, the tip **112** can contact, and form a seal with, the inner wall of a blood vessel. The tip **112** can seal against the vessel wall proximal of the clot **106** when deployed to the expanded configuration. After removal of the dilator **110,** the clot **106** can then be extracted via aspiration by the device **100.**

The funnel catheter **104** can be constructed from a shape memory polymer form. The shape memory polymer form can incorporate support webs or stints of the same material or a second polymer. The funnel catheter **104** can be composed of an elastic membrane that is overcome by an encapsulated shape memory metallic frame, wherein the frame may be formed of a braided polymer, spring steel, NiTi wire, laser cut shape memory metal tubing such as NiTi that can be expanded and heat-set in a desired shape, or spring steel formed and then annealed in a desired shape. The funnel catheter **104** can be composed of a floating elastic membrane that is supported by an inner shape memory metallic frame, wherein the frame may be formed by a braided spring steel or NiTi wire, laser cut shape memory metal tubing such as NiTi that can be expanded and heat set in a desired shape, or spring steel formed and annealed in a desired shape.

Additional materials may also be incorporated into the funnel catheter **104,** such as DFT wire to provide radiopacity. In examples of the funnel catheter **104** including NiTi, the NiTi strands may be coated or treated to adhere to, chemically bond to, or impregnate a radiopaque outer later such as gold. In examples of the funnel catheter **104** composed of polymer braids, polymer braids such as PET or PEEK may be braided alongside each other or with metallic wire counterparts such as NiTi, spring steel, platinum, or DFT.

The funnel catheter **104** can include lasered hypotubing, braiding, and/or multiple polymer lamination layers formed in segments or via continuous blending of polymers with different hardnesses, which can to enhance the torqueability, pushability, flexibility, and kink resistance of the funnel catheter **104.** The funnel shape formed by the tip **112** when expanded can improve aspiration efficiency, reduce friction, and lessen the risk of vessel trauma from snagging on vessel openings. The maximum diameter **D1** of the tip **112** can be larger than the diameter of the target blood vessel. The diameter of a target blood vessel can range from approximately 3.2mm-5.5mm.

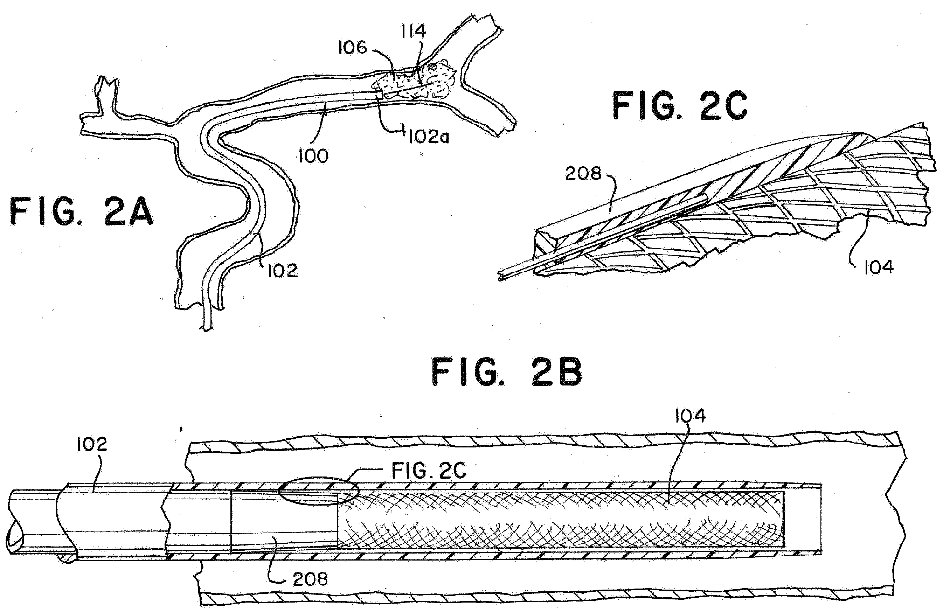

In Fig. 2a, similar to Fig. 1a, there is illustrated a device **100** for removing an occlusive clot **106** from a vessel of a patient according to this disclosure. The device **100** can have an outer sheath layer **102** facilitating the introduction of microcatheters, funnel catheters, guidewires, or any of a number of other products to a target site within the vasculature. The outer sheath **102** can have a distal end **102a,** a proximal end located outside a patient, and an internal lumen extending proximal of the distal end **102a** and terminating within the proximal end. The outer sheath **102** can be positioned within the vasculature of a patient so that the distal end **102a** is proximate a targeted occlusive clot **106.**

In Fig. 2b, the funnel catheter **104** is shown within the outer sheath **102** in the collapsed configuration as the device **100** is positioned proximate a target clot location within a blood vessel. The funnel catheter **104** can also be at least partially enclosed within a funnel sheath **208** within the outer sheath **102,** whereby at least a portion of the distal end **104a** of the funnel catheter **104** can extend distally into the outer sheath **102** from the funnel sheath **208.** The funnel sheath **208** can provide stability and compression to the funnel catheter **104** as it is advanced through the outer sheath **102** while leaving at least a portion of the distal end **104a** uncovered to allow for the distal tip **112** to expand inside the blood vessel upon deployment. The funnel catheter **104** with funnel sheath **208** and the outer sheath **102** can be provided as standalone components to be used in conjunction with one another.

The funnel sheath **208** can be constructed using techniques commonly known in the art such as braiding, laminating, or extruding. Axial support wire may be included between the braid or lamination layers of the funnel sheath **208.** As shown in Fig. 2c, a close-up cross sectional view of the funnel sheath **208,** axial support wire may also be threaded through the braid construction of the funnel sheath **208.**

The funnel catheter **104** with funnel sheath **208** can be placed into to the outer sheath **102** prior to deployment of the device **100** to a target site. The funnel catheter **104** with funnel sheath can be advanced into the outer sheath **102** using a tool such as a removable split sheath by retracting and collapsing the funnel catheter **104** with funnel sheath **208** into the split sheath, inserting the split sheath enclosing the funnel catheter **104** into the proximal end of the outer sheath **102,** advancing the funnel catheter **104** with funnel sheath **208** distally into the outer sheath **102,** and removing the split sheath shaft. The split sheath shaft can be reattached to the funnel catheter **104** with funnel sheath **208** should an operator desire to add length to the funnel catheter **104** or make multiple passes with the funnel catheter **104.** Similar to Figs. 1a-1e, the funnel catheter **104** may be manufactured using shape memory metals or polymers, laser cut metals, or polymer tubes, which can be expanded and heat set to revert to a funnel shape when advanced past the distal tip **102a** of the outer sheath **102.**

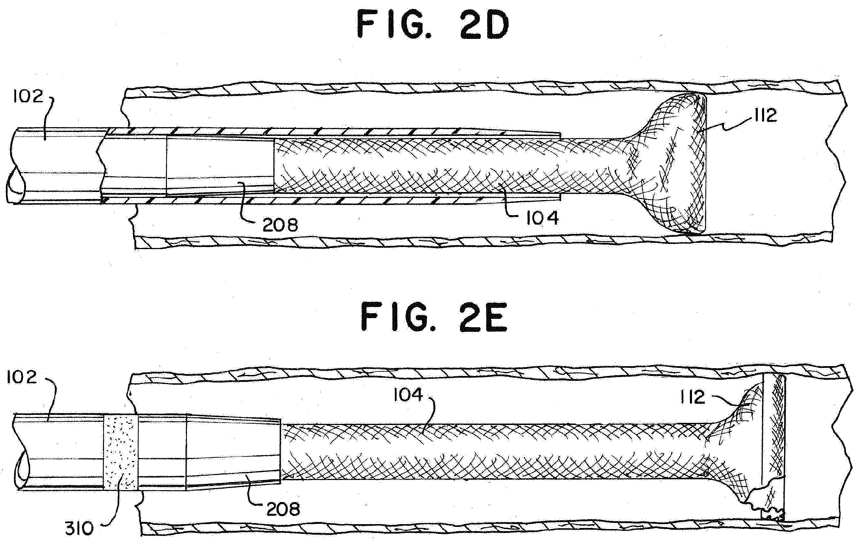

As shown in Fig. 2d, once the device is in position proximate the target location, the outer sheath **102** can be retracted proximally to expose the funnel catheter **104.** The distal tip **112** can then radially expand to seal the vessel in preparation for aspirating a clot. The distal tip **112** can include an encapsulated membrane composed of elastic, flexible, or porous material. The tip **112** can thus seal with the vessel or create enough of a restriction such that when aspiration is applied, blood and the clot distal of the mouth will be drawn into the funnel catheter **104** rather than blood proximal of the tip **112.** If the expanded tip **112** does not seal, or no other seal exists between the funnel catheter **104** and the inner wall of the blood vessel, then the suction applied to the clot can be ineffective as the less restricted flow proximal of the tip **112** would dominate.

In Fig. 2e, the funnel catheter **104** can also be advanced distally from the outer sheath **102** to maintain a better seal with the blood vessel or create more efficient access to a clot. The distal tip **112** of the funnel catheter **104** can be atraumatic, allowing the funnel catheter **104** to be advanced distally without injuring the blood vessel. The distal tip **112** can include a lubricous polymer rib that allows the funnel tip to glide easily through blood vessels. As shown in Fig. 2e, the distal tip **112** can invert with low force during advancement to avoid injuring the blood vessel, and revert to its funnel shape when retracted. Once the distal tip **112** is deployed and appropriately positioned, the clot can be aspirated.

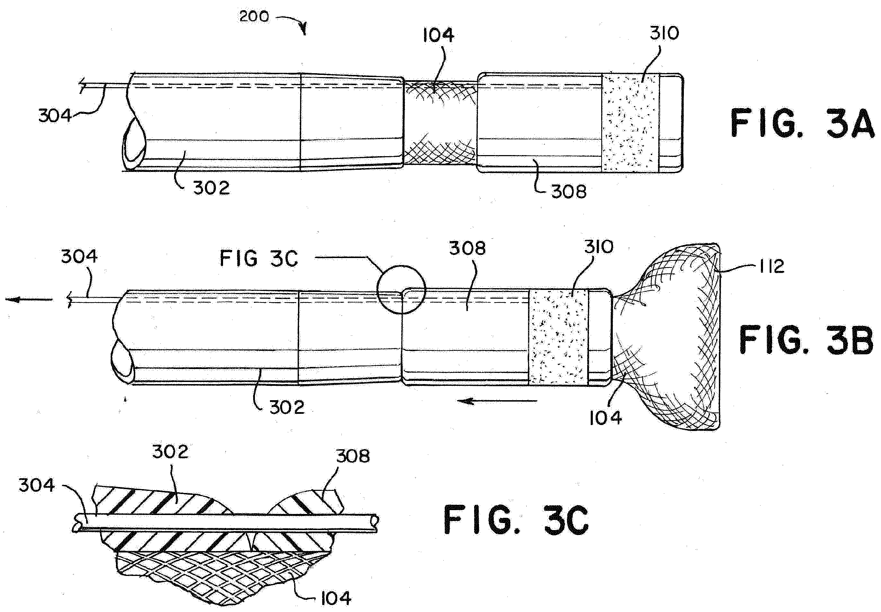

Fig. 3a illustrates an alternate distal end **104a** of a funnel catheter **104** in a device **200** for clot removal via aspiration, the device **200** having an alternate mechanism for funnel catheter **104** actuation via a pull wire **304.** Pull wire actuation may be provided for the funnel catheter **104** so that no additional outer or inner catheter is required to assist with expanding or collapsing the distal tip **112.** Accordingly, the device **200** as shown in Fig. 3a does not require an outer sheath **102** as in Figs. 1 and 2 to expand or collapse the distal tip **112,** although an outer sheath **102** may still be utilized for delivery of the device **200** to the treatment site. Rather, the funnel catheter **104** can have an outer lamination layer **302** covering at least a portion of the funnel catheter **104** and with openings formed in the layer **302** that allow translation of one or more pull wires **304.** The one or more pull wires **304** may be supplied in a rapid exchange format. To maximize flexibility at the distal tip **112,** the one or more pull wires **304** can be threadlike as to not stiffen or increase the rigidity of the distal tip **112.** The funnel catheter **104** can also have a floating retaining sheath **308** attached to the catheter **104** via the one or more pull wires **304.** The floating retaining sheath **308** can contain a radiopaque marker band **310.** The radiopaque marker band **310** can include proximal coils to maintain flexibility of the distal end **104a** of the funnel catheter **104** upon delivery to and deployment at a target site while aiding in radial retention of the funnel catheter **104** in the collapsed configuration. The proximal coils can be approximately 0.001"-0.002" in diameter. (1 inch ≈ 2.54 cm). Radiopaque coils provide greater flexibility compared to other radiopaque marker bands, which assist with the tracking of the distal end **104a** of the funnel catheter **104.** The funnel catheter **104** can be collapsed into the collapsed configuration outside the body by hand or by use of ancillary medical devices prior to delivery to a treatment site.

In Fig. 3b, the distal tip **112** can be expanded to the deployed configuration by utilizing the one or more pull wires to shift the floating retaining sheath **308** proximally towards the outer lamination layer **302,** thereby exposing the distal tip **112** and allowing it to expand radially outward to its predetermined diameter as discussed in Figs. 1a-1e. The distal tip **112** can be atraumatic to the vessel wall upon expansion. Once the distal tip **112** is deployed and appropriately positioned, the clot can be aspirated.

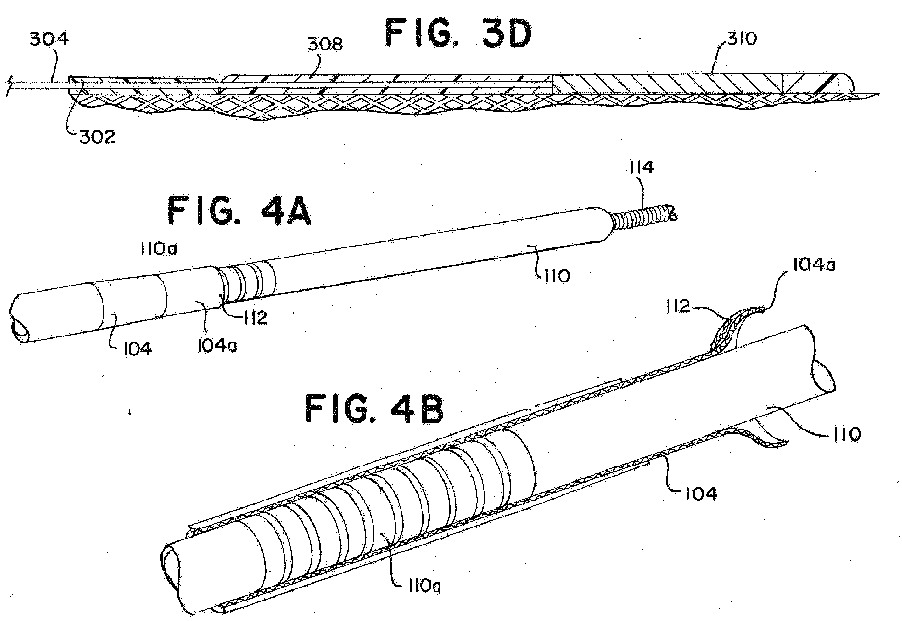

In Figs. 3c and 3d, expanded views of an example pull wire **304** connecting the lamination layer **302** to the floating retaining sheath **308** are shown. The one or more pull wires **304** can be stiff enough to allow distal advancement of the floating retaining sheath **308** to return the distal tip **112** to the collapsed configuration for resheathing or removal of the device **200.** This can be accomplished by advancing the floating retaining sheath **308** distally over the distal tip **112** manually by hand, with the aid of an ancillary medical device or loading tool, or by advancing the one or more pull wires **304** distally. The device may contain additional features to prevent distal movement of the floating retaining sheath **308** beyond the distal tip **112** of the funnel catheter **104.** The distal tip **112** can be retracted proximally into an outer sheath **102** for removal or relocation at a location proximal to the treatment location.

Figs. 4a-4b depict a device **100** wherein the funnel catheter **104** can contain ferromagnetic material at the distal end **104a,** including at the distal tip **112,** and the funnel catheter **104** can be expanded to the deployed state or retracted to the collapsed state by passing over an inner dilator catheter **110** wherein at least a portion of the inner dilator catheter **110** can have magnetic properties **110a.** The inner dilator catheter **110** can be at least partially enclosed within the funnel catheter **104.** The funnel catheter **104** and the dilator **110** can be delivered to a target treatment site over a guidewire **114.**

The one or more magnetic portions **110a** of the inner dilator catheter **110** can include magnetic rings formed into the dilator shaft. The rings can have an inner diameter of approximately 0.050" or less. The magnetic rings can have an outer diameter of approximately 0.065" or less. (1 inch ≈ 2.54 cm). The magnetic rings can be covered with a thin film to isolate metals utilized in the magnets from oxygen to prevent rusting. Magnets utilized in the inner dilator catheter **110** may be anisometric or isometric. Magnets used in the inner dilator catheter **110** can be neodymium magnets. Alternatively, the funnel catheter **104** can have magnetic properties, and the dilator shaft **110** can include at least one ferromagnetic portion. Electromagnets can also be incorporated into the funnel catheter **104** or dilator catheter **110.**

In Fig. 4a, the funnel catheter **104** is in the collapsed form for delivery to a treatment site. When the ferromagnetic distal end **104a** passes over a magnetic portion **110a** of the inner dilator catheter **110,** the ferromagnetic material at the distal end **104a** is attracted to the magnetic portion **110a** of the inner dilator catheter **110** which retracts the funnel catheter **104** into the collapsed configuration. The funnel catheter **104** in the collapsed configuration can be more easily delivered to a target site or maneuvered within a blood vessel.

The ferromagnetic material of the distal end **104a** can be a stainless-steel funnel support structure with low to zero nickel content. The distal end **104a** can be formed in a predisposed open position and encapsulated with a highly elastic membrane. When the distal tip **112** expands in the deployed configuration, the membrane can be stretched to assist in occluding the blood vessel. Alternatively, the distal end **104a** can be enclosed by a relatively loose or baggy membrane not under tension.

In Fig. 4b, the retaining force between the ferromagnetic funnel and magnetic dilator is removed by retracting the dilator **110** proximally relative to the funnel catheter **104,** which can break the alignment between the ferromagnetic distal end **104a** and the magnetic portion of the dilator **110a**. This allows the distal end **112** to expand to the deployed funnel configuration. Once the distal tip **112** is deployed and appropriately positioned, the clot can be aspirated.

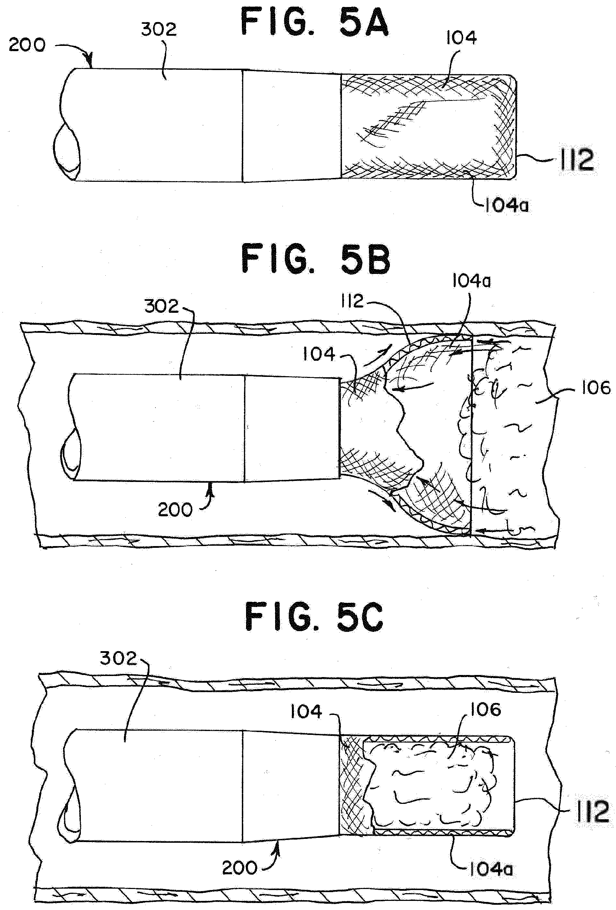

Figs. 5a-5c depict a device **200** wherein the funnel catheter **104** is not predisposed to an expanded deployed configuration. Rather, the funnel catheter **104** can be supplied to a target site in predisposed collapsed configuration for low-profile atraumatic delivery to the target site. In Fig. 5a, the distal tip **112** can have a different braid construction than other portions of the braid proximal to the tip **112.** The distal tip **112** can have a wider braid angle and/or a lower pics-per-inch (PPI) than areas of the funnel catheter **104** proximal to the tip **112.** (1 inch ≈ 2.54 cm). A wide braid angle can allow space for the funnel catheter **104** to compress under aspiration such that the braid angle reduces upon expansion of the distal tip **112.** The distal tip **112** can be encapsulated or covered with a highly elastic material or membrane. The portions of the funnel catheter proximal to the distal tip **112** can be at least partially covered by at least one layer of lamination **302** to make said portions more stiff in comparison to the distal tip **112.**

In Fig. 5b, when aspiration is applied to the proximal end of the catheter, a negative flow is achieved at the distal tip **112.** The distal tip **112** can be compressed proximally as a result of the compressive forces applied through negative flow. As a result, the diameter of the distal tip **112** can increase upon proximal compression and the membrane stretched over the tip **112** can expand as well. The distal tip **112** can expand to contact the vessel walls at the target site, sealing the vessel and forming a funnel mouth that can receive a clot **106** during aspiration.

When aspiration flow is stopped or removed, the shape memory of the elastic membrane and/or distal tip **112** can return to the collapsed configuration. When the distal tip **112** returns to the collapsed configuration, it can grasp and retain any portions of the clot **106** lodged therein, as depicted in Fig. 5c.

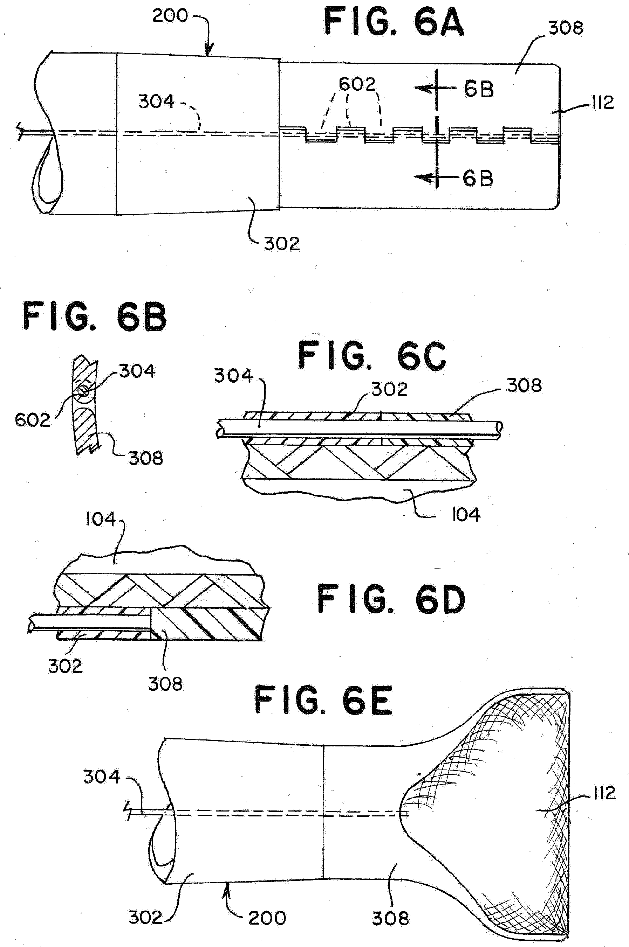

Figs. 6a-6c depict a device **200** wherein a zip cover actuation and a pull wire are utilized to expand the distal tip to the deployed state for clot removal. In Fig 6a, a pull wire system can be used similar to the one described in Figs. 3a-3c. The device **200** can have a funnel catheter **104,** predisposed to expand outwardly at the distal tip **112** upon deployment, with an outer lamination layer **302** covering at least a portion of the funnel catheter **104** and with openings formed in the layer that allow translation of one or more pull wires **304.** The funnel catheter **104** can also have a retaining sheath **308** attached to the catheter **104** via the one or more pull wires **304.** The retaining sheath can include a longitudinal separation. The longitudinal separation can have a series of openings **602** on either side of the separation, the openings **602** being sequentially aligned longitudinally. In other words, openings **602** can be sequential interlocking tabs or rectangular edges whereby an inwardly extended edge can be sequentially followed by an outwardly extended edge, and so on until terminating at the distal tip 112. The pull wire **304** can extend through the openings **602,** holding the retaining sheath in a configuration to maintain the distal tip **112** in the collapsed configuration. Alternatively, the retaining sheath **308** can include a zip-lock type fastening to which the pull wire **304** is attached. The pull wire **304** can have an abutment at the distal end of the wire that requires a threshold amount of force to retract the pull wire **304** fully to facilitate expansion of the distal tip **112** in order to prevent inadvertent uncovering during advancement of the device **200** through the vasculature.

Depicted in Figs. 6b and 6c are expanded views of the pull wire **304** running from the outer lamination layer **302** through the longitudinal interlocking openings **602** of the retaining sheath **308.** Fig. 6d depicts a longitudinal support wire in the lamination layer that does not run through the retaining sheath **308.**

Fig. 6e depicts the device wherein the distal tip **112** is in the deployed configuration. The pull wire **304** can be retracted proximally to break the longitudinal connections of the retaining sheath **308,** allowing the distal tip **112** to expand. Once the distal tip **112** is deployed and appropriately positioned, a clot can be aspirated.

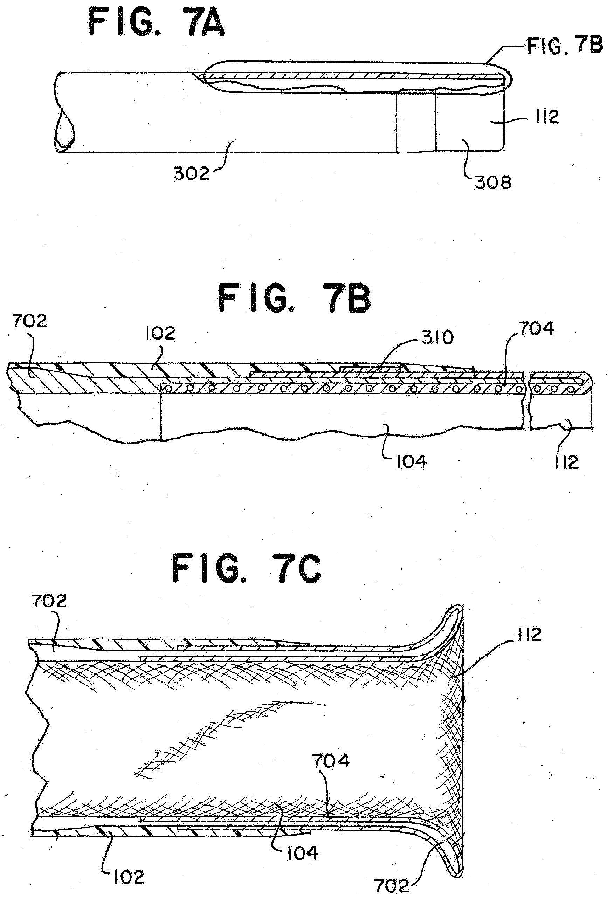

Figs. 7a-7c depict an example of the device **100** wherein the funnel catheter **104** is at least partially encapsulated by an outer sheath **102** and can expand radially upon deployment due to one or more inner support structures **702** that can flare radially outward when the outer sheath **102** is retracted proximally relative to the funnel catheter **104.** As depicted in Fig. 7a and expanded view Fig. 7b, the device **100** can be collapsed into a collapsed configuration for delivery to a target treatment site. The device **100** can have a funnel catheter **104.** The funnel catheter **104** can be composed of braided materials, laser cut tubing, ribbed polymers, multi-layered polymers, or composite polymers. The distal tip **112** of the funnel catheter **104** can be at least partially covered by an encapsulated or floating membrane **704** to obstruct blood flow upon deployment. The funnel catheter **104** can have one or more internal support structures **702.** The one or more inner support structures **702** can be porous to partially restrict blood flow. The device **100** an include an outer sheath **102** at least partially enclosing the funnel catheter **104.** The outer sheath **102** can be elastic.

As depicted in Fig. 7c, when the outer sheath **102** is retracted proximally relative to the funnel catheter **104,** the one or more internal support structures **702** in the funnel catheter can flare radially outward towards a vessel wall. The flaring of the internal support structures **702** can expand the distal tip **112** to atraumatically contact the vessel wall, creating a funnel for clot aspiration. Once the distal tip **112** is deployed and appropriately positioned, the clot can be aspirated as described in Fig. 1e. The retraction force on the outer sheath **102** can later be released to move the outer sheath **102** distally relative to the funnel catheter **104,** re-covering the distal tip **112** and collapsing the distal tip **112** back into the collapsed configuration for removal from the vasculature or further advancement into the vasculature of the patient.

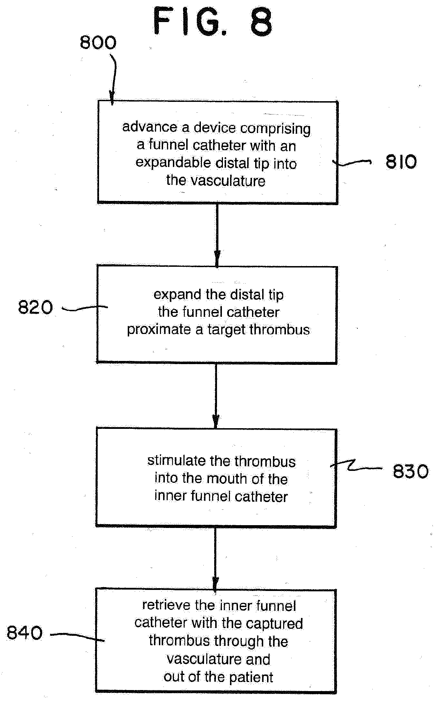

Fig. 8 depicts a flow diagram outlining a method of use of the device according to aspects of the present disclosure. The method can have some or all of the following steps and variations thereof, and the steps are recited in no particular order. The method can have the steps of advancing a device comprising a funnel catheter with an expandable distal tip into the vasculature **810;** expanding the distal tip of the funnel catheter proximate a target thrombus **820;** stimulating the thrombus into the mouth of the funnel catheter **830;** and retrieving the funnel catheter with the captured thrombus through the vasculature and out of the patient **840.**

When delivered to the target site, the distal tip **112** of the funnel catheter **104** can be deployed to expand radially in order to contact the inner walls of the blood vessel. The profile of the tip can seal against the vessel wall proximal of the target site. This seals off vessel fluid proximal to the mouth and provides a large opening to easily receive the clot. For this reason, the method may further include the step of coating the expandable distal tip with a membrane.

The step of advancing a device comprising a funnel catheter with an expandable distal tip into the vasculature can further comprise use of an outer sheath to advance the funnel catheter.

Where the device referenced in step 810 further comprises a funnel retaining sheath and a dilator, the step of expanding the distal tip of the funnel catheter proximate a target thrombus can further displacing the dilator relatively distally of the funnel catheter.

Where the device referenced in step 810 further comprises an outer sheath, the step of expanding the distal tip of the funnel catheter proximate a target thrombus can further comprise retracting the outer sheath proximally to expose the funnel catheter.

Where the device referenced in step 810 further comprises an outer lamination layer, one or more pull wires, and retaining sheath, the step of expanding the distal tip of the funnel catheter proximate a target thrombus can further comprise utilizing the one or more pull wires to shift the retaining sheath proximally towards the outer lamination layer to expose the distal tip.

Where the device referenced in step 810 further comprises an dilator with magnet properties and ferromagnetic material in at least a portion of the funnel catheter, the step of expanding the distal tip of the funnel catheter proximate a target thrombus can further comprise retracting the dilator proximally relative to the funnel catheter to break the alignment between the ferromagnetic distal tip and the magnetic portion of the dilator.

Where the expandable distal tip referenced in step 810 has a wider braid angle relative to the proximal portions of the funnel catheter, the step of expanding the distal tip of the funnel catheter proximate a target thrombus can further comprise applying aspiration to a proximal end of the funnel catheter to achieve a negative flow at the distal tip.

Where the device referenced in step 810 further comprises an outer lamination layer, one or more pull wires, and a retaining sheath comprising one or more longitudinal interlocking openings, the step of expanding the distal tip of the funnel catheter proximate a target thrombus can further comprise retracting the pull wire proximally to break the interlocking openings of the retaining sheath.

The step of stimulating the thrombus into the mouth of the clot retrieval catheter can comprise using aspiration, thrombectomy devices, or other microcatheter or medical devices known in the art.

The disclosure is not limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near to or a direction towards the physician.

In describing examples, terminology is resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method can be performed in a different order than those described herein without departing from the scope of the disclosed technology. Similarly, it is also to be understood that the mention of one or more components in a device or system does not preclude the presence of additional components or intervening components between those components expressly identified.

As discussed herein, a "patient" or "subject" can be a human or any animal. It should be appreciated that an animal can be a variety of any applicable type, including, but not limited to, mammal, veterinarian animal, livestock animal or pet-type animal, etc. As an example, the animal can be a laboratory animal specifically selected to have certain characteristics similar to a human (e.g., rat, dog, pig, monkey, or the like).

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

By "comprising" or "containing" or "including" is meant that at least the named compound, element, particle, or method step is present in the composition or article or method, but does not exclude the presence of other compounds, materials, particles, method steps, even if the other such compounds, material, particles, method steps have the same function as what is named.

It must also be noted that, as used in the specification and the appended claims, the singular forms "a," "an," and "the" include plural referents unless the context clearly dictates otherwise. Ranges can be expressed herein as from "about" or "approximately" one particular value and/or to "about" or "approximately" another particular value. When such a range is expressed, other exemplary embodiments include from the one particular value and/or to the other particular value.

The descriptions contained herein are examples of the disclosure and are not intended in any way to limit the scope of the disclosure. While particular examples of the present disclosure are described, various modifications to devices and methods can be made without departing from the scope of the disclosure. For example, while the examples described herein refer to particular components, the disclosure includes other examples utilizing various combinations of components to achieve a described functionality, utilizing alternative materials to achieve a described functionality, combining components from the various examples, combining components from the various example with known components, etc. The disclosure contemplates substitutions of component parts illustrated herein with other well-known and commercially-available products. The invention is defined by the claims which follow.

## Claims

1. A device (200) to retrieve an obstruction in a blood vessel, comprising:
a funnel catheter (104) comprising an expanding distal tip (112), a distal end located at the distal tip (104a), and a proximal portion; **characterised by**
an outer lamination layer (302) covering at least a portion of the funnel catheter (104) wherein the outer lamination layer (302) further comprises openings in the layer to allow translation of one or more pull wires (304); and
a floating retaining sheath (308) attached to the funnel catheter (104) via the one or more pull wires (304).

2. The device (200) of claim 1 wherein i) the funnel catheter has a braid construction and the distal tip (112) has a wider braid angle relative to the proximal portion of the funnel catheter, ii) the funnel catheter has a braid construction and the distal tip (112) has a lower number of pics-per-inch (PPI) relative to the proximal portion of the funnel catheter, or iii) the distal tip (112) is at least partially covered with an elastic membrane (1 inch ≈ 2.54 cm).

3. The device (200) of claim 1 wherein the one or more pull wires (304) extend through one or more longitudinal interlocking openings (602) in the retaining sheath (308).

4. The device (200) of claim 1 wherein the retaining sheath (308) further comprises a zip-lock type fastening to which the one or more pull wires (304) are attached.

## Patentansprüche

1. Vorrichtung (200), um eine Obstruktion in einem Blutgefäß zu bergen, umfassend:
einen Trichterkatheter (104), umfassend eine sich erweiternde distale Spitze (112), ein distales Ende, das sich an der distalen Spitze (104a) befindet, und einen proximalen Abschnitt; **gekennzeichnet durch**
eine äußere Laminierungsschicht (302), die mindestens einen Abschnitt des Trichterkatheters (104) bedeckt, wobei die äußere Laminierungsschicht (302) ferner Öffnungen in der Schicht umfasst, um eine Translation eines oder mehrerer Zugdrähte (304) zu ermöglichen; und
eine schwebende Haltehülle (308), die an dem Trichterkatheter (104) über den einen oder die mehreren Zugdrähte (304) befestigt ist.

2. Vorrichtung (200) nach Anspruch 1, wobei i) der Trichterkatheter eine geflochtene Konstruktion aufweist und die distale Spitze (112) einen breiteren Flechtwinkel relativ zu dem proximalen Abschnitt des Trichterkatheters aufweist, ii) der Trichterkatheter eine geflochtene Konstruktion aufweist und die distale Spitze (112) eine geringere Anzahl von Pics pro Zoll (PPI) relativ zu dem proximalen Abschnitt des Trichterkatheters aufweist, oder iii) die distale Spitze (112) mindestens teilweise mit einer elastischen Membran (1 Zoll ≈ 2,54 cm) bedeckt ist.

3. Vorrichtung (200) nach Anspruch 1, wobei sich der eine oder die mehreren Zugdrähte (304) durch eine oder mehrere längs verlaufende Verriegelungsöffnungen (602) in der Haltehülle (308) erstrecken.

4. Vorrichtung (200) nach Anspruch 1, wobei die Haltehülle (308) ferner eine reißverschlussartige Fixierung umfasst, an der der eine oder die mehreren Zugdrähte (304) befestigt sind.

## Revendications

1. Dispositif (200) de retrait d'une obstruction dans un vaisseau sanguin, comprenant :
un cathéter en entonnoir (104) comprenant une pointe distale en expansion (112), une extrémité distale située au niveau de la pointe distale (104a), et une partie proximale ; **caractérisé par**
une couche de stratification externe (302) recouvrant au moins une partie du cathéter en entonnoir (104) dans lequel la couche de stratification externe (302) comprend en outre des ouvertures dans la couche pour permettre une translation d'un ou plusieurs fils de traction (304) ; et
une gaine de retenue flottante (308) attachée au cathéter en entonnoir (104) par l'intermédiaire du ou des fils de traction (304).

2. Dispositif (200) selon la revendication 1, dans lequel i) le cathéter en entonnoir a une construction tressée et la pointe distale (112) a un angle de tressage plus large par rapport à la partie proximale du cathéter en entonnoir, ii) le cathéter en entonnoir a une construction tressée et la pointe distale (112) a un nombre inférieur de duites par pouce (PPI) par rapport à la partie proximale du cathéter en entonnoir, ou iii) la pointe distale (112) est au moins partiellement recouverte d'une membrane élastique (1 pouce ≈ 2,54 cm).

3. Dispositif (200) selon la revendication 1, dans lequel le ou les fils de traction (304) s'étendent à travers une ou plusieurs ouvertures d'enclenchement réciproque longitudinal (602) dans la gaine de retenue (308).

4. Dispositif (200) selon la revendication 1, dans lequel la gaine de retenue (308) comprend en outre une fermeture de type par pression et glissière à laquelle sont attachés le ou les fils de traction (304).
